# EUROPEAN PATENT SPECIFICATION

(11) **EP 1 858 836 B1**
(45) Date of publication and mention of the grant of the patent: **18.11.2009**
(21) Application number: 06710103.0
(22) Date of filing: 16.03.2006
(51) Int. Cl.: C07C 69/94, C07D 303/46, C07D 405/12, C07D 211/38, C07D 211/42, A61K 31/4523, A61P 35/00

(54) **ANALOGUES OF THE AZINOMYCINS AS ANTI-TUMOUR AGENTS AND AS PRODRUGS**
ANALOGA DER AZINOMYCINE ALS ANTITUMORMITTEL UND PRODRUGS
ANALOGUES DES AZINOMYCINES COMME AGENTS ANTI-TUMEUR ET PROMEDICAMENTS

(30) Priority: 18.03.2005 GB 0505644
(43) Date of publication of application: 28.11.2007
(73) Proprietor: University of Bradford, Bradford BD7 1DP (GB)
(72) Inventor: SCARCEY, Mark, Norwich, NR4 7TJ (GB); PATTERSON, Laurence Hylton Inst. of Cancer Therap., West Yorkshire BD7 1DP (GB); PORS, Klaus Inst. of Cancer Therap., West Yorkshire BD7 1DP (GB); CASELY-HAYFORD Maxwell, Lord's Wood, Chatham ME5 8TW (GB)
(74) Representative: O'Neill, Michelle
(86) International application number: PCT/GB2006/000941
(87) International publication number: WO 2006/097730

(56) References cited:
- DATABASE CROSSFIRE BEILSTEIN BEILSTEIN INTITUT ZUR FOERDERUNG DER CHEMISCHEN WISSENSCHAFTEN, FRANKFURT AM MAIN, DE; XP002363741 Database accession no. 117694 & BERMANN, GRAFE: HOPPE-SEYLER'S Z. PHYSIOL. CHEM., vol. 187, 1930, page 193,
- DATABASE CROSSFIRE BEILSTEIN BEILSTEIN INTITUT ZUR FOERDERUNG DER CHEMISCHEN WISSENSCHAFTEN, FRANKFURT AM MAIN, DE; XP002363742 Database accession no. 1763663 & RAMBAUD: BULL. SOC. CHIM. FR., vol. 5, no. 1, 1934, page 1335,
- DATABASE CROSSFIRE BEILSTEIN BEILSTEIN INTITUT ZUR FOERDERUNG DER CHEMISCHEN WISSENSCHAFTEN, FRANKFURT AM MAIN, DE; XP002363743 Database accession no. 1772855 & RAMBAUD: BULL. SOC. CHIM. FR., vol. 5, no. 1, 1934, page 1317,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363744 Database accession no. 1775745 & RAMBAUD: BULL. SOC. CHIM. FR., vol. 5, no. 1, 1934, page 1317,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363745 Database accession no. 5014296 & BRETSCHEIDER ET AL.: TETRAHEDRON, vol. 44, no. 17, 1988, pages 5403-5414,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363746 Database accession no. 5065689 & GUAY ET AL.: SYNTHESIS, vol. 3, 1987, pages 294-297,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363747 Database accession no. 5116994 & WADE ET AL.: TETRAHEDRON, vol. 40, no. 3, 1984, pages 601-612,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363748 Database accession no. 8316184 & SYNTHESIS, 1999, pages 1399-1400,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363749 Database accession no. 8328144 & SYNTHESIS, 1999, pages 1399-1400,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363750 Database accession no. 8487778 & CLARK ET AL.: J. CHEM. SOC. PERKIN TRANS. 1, vol. 7, 2000, pages 1117-1128,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363751 Database accession no. 8545821 & BERKOWITZ ET AL.: J. ORG. CHEM., vol. 65, no. 10, 2000, pages 2907-2918,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363752 Database accession no. 1923363 & ENGER ET AL.: BULL. SOC. CHIM. FR., 1975, pages 1681-1685,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363753 Database accession no. 1866957 & THUAN ET AL.: TETRAHEDRON, vol. 34, 1978, pages 1469-1474,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363754 Database accession no. 1762175 & RAMBAUD ET AL.: HEBD. SEANCES ACAD. SCI., vol. 223, 1946, page 381,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363755 Database accession no. 1762168 & RAMBAUD ET AL.: HEBD. SEANCES ACAD. SCI., vol. 223, 1946, page 381,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363756 Database accession no. 1756573 & RAMBAUD ET AL.: BULL. SOC. CHIM. FR., vol. 5, no. 1, 1934, page 1317,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363757 Database accession no. 1754029 & VOGEL ET AL.: HELV. CHI. ACTA, vol. 33, 1950, page 116,
- DATABASE CROSSFIRE BEILSTEIN BEILSTEIN INTITUT ZUR FOERDERUNG DER CHEMISCHEN WISSENSCHAFTEN, FRANKFURT AM MAIN,; XP002363758 Database accession no. 1753697 & VAN DER SLEEN ET AL.: RECL. TRAV. CHIM. PAYS-BAS, vol. 21, 1902, page 227,
- DATABASE CAPLUS CHEMICAL ABSTRACTS SERVICE, COLUMBUS, OHIO, US; XP002363759 Database accession no. 1973:84781 & BUDEAUNU ET AL.: ANALELE STIINTIFICE ALE UNIVERSITATII AL. I. CUZA DIN IASI SECTIUNEA 1C: CHIMIE, vol. 18, no. 2, 1972, pages 169-181,
- DATABASE CAPLUS CHEMICAL ABSTRACTS SERVICE, COLUMBUS, OHIO, US; XP002363760 Database accession no. 1974:421113 & BUDEANU ET AL.: REVISTA MEDICO-CHIRURGICALA, vol. 77, no. 4, 1973,
- DATABASE CAPLUS CHEMICAL ABSTRACTS SERVICE, COLUMBUS, OHIO, US; XP002363761 Database accession no. 1985:95348 & MANOLOV ET AL.: DOKLADY BOLGARSKOI AKADEMII NAUK, vol. 37, no. 9, 1974,
- DATABASE CAPLUS CHEMICAL ABSTRACTS SERVICE, COLUMBUS, OHIO, US; XP002363762 Database accession no. 1980:639862 & BUDEAUNU ET AL.: FARMACIA (BUCHAREST, ROMANIA), vol. 28, no. 1, 1980, pages 1-5,
- CASELY-HYFORD ET AL.: "Truncated azinomycin analogues intercalate into DNA" BIOORG. MED. CHEM. LETT., vol. 15, 2005, pages 653-656,

## Description

The present invention concerns aromatic oxidation-activated prodrugs, particularly anti-tumour prodrugs and those which are activated by the oxidation activities of the cytochrome P450 family of enzymes. The prodrugs may be alkylating agents having topoisomerase II inhibiting activities.

Many conventional cytotoxic drugs are known that can be used for therapeutic purposes. However, they typically suffer from the problem that they are generally cytotoxic and therefore may affect cells other than those that are required to be destroyed. This can be alleviated to some extent by the use of targeted drug delivery systems, for example direct injection to a site of tumourous tissue or, e.g. binding the cytotoxic agent to an antibody that specifically recognises an antigen displayed only on the cancer cell surface. Alternatively, electromagnetic radiation my be used to cause chemical alteration in an agent at a desired site such that it becomes cytotoxic. However, all of these techniques have, to a greater or lesser extent, certain limitations and disadvantages.

The azinomycins A and B are potent anti-tumour agents that bind to DNA by alkylation in the major groove and lead to cell death. However, they are relatively unstable, have poor availability from natural sources and are unlikely to proceed into the clinic.

These naturally occurring compounds, along with the truncated analogue A (see structure below), were first isolated from Streptomyces griseofuscus S42227 by Nagaoka et al in Japan (J. Antibiot. (Tokyo) 1986, 39, 1527-1532).

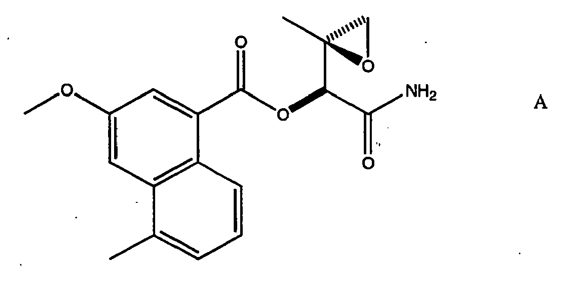

Armstrong in Tetrahedron Lett. 1991,32,3807-3810 later disclosed using mass and NMR special data, that the anti-tumour antibiotic carzinophilin, isolated in 1954 from Streptomyces sahachiror (Onda et al, J. Antibiot. 1969, 22, 42-44) was the same compound as natural product azinomycin B.

Shibuya in Tetrahedron Lett. 1983,24,1175-1178 describes the first synthetic studies of the azinomycins but these are inaccurate as they were based upon the erroneous structure of carzinophilin suggested by Lain et al in J. Am. Chem. Soc.1982, 104, 3213-3214.

Truncated analogue A was first correctly synthesized by Shibuya et al in Tetrahedron Lett. 1987,28,2619-2622 where the commercially available diacetone D-glucose from the chiral pool was used in a lengthy multistep synthesis to stereospecifically generate the analogue A, of the structure shown above, with the same stereochemistry as the natural products.

The majority of other studies on the epoxide fragment of the azinomycins and on the synthesis of A have focused on the use of Sharpless asymmetric epoxidation. Direct efforts on synthesising enantiopure precursors are described by Konda et al in Chem. Pharmac. Bull 1994, 42, 285-288. Shipman et al in Chem.Soc. Perkin Trans. 11998,1249-1255 further discuss a Sharpless asymmetric dihydroxylation/asymmetric epoxidation methodology to give the required S,S isomer in excellent yield.

Both Armstrong et al (J. Am. Chem. Soc. 1992,114, 371-372) and Coleman et al (J. Org. Chem.1992, 57,5813-5815) have independently described synthetic routes to the aziridine core of Azinomycin A. The total synthesis proved more elusive and has only recently been described by Coleman et al (Angew. Chem. Int. Ed. 2001, 491736-1739). The key to the total synthesis was assembly of the backbone of the natural product, including the epoxide moiety, followed by the late stage introduction of the azabicyclic system through a Wadsworth-Horner-Emmons reaction.

The synthesis of the left-hand fragment of the azinomycins allowed the study of its interactions with DNA. Zang et al in Biochemistry 2000, 39,14968-14975 present data to suggest that structure A intercalates with DNA via its naphthalene subunit and alkylates guanine residues at N7 with little, if any sequence selectivity. Shipman et al used these findings in structure-activity surveys to identify analogues of the natural products that might be useful as anti-tumour agents. (Bioorg. Med. Chem. Lett 2000, 10, 239-241). Replacement of the 3-methoxy-5-methylnaphthalene with a phenyl group (which would be expected to show little affinity for DNA through intercalation) effectively removed the biological potency of the epoxide in a variety of cell lines. In Chem. Commun. 2000, 2325-2326 Hortley et al study the DNA cross-linking activity of symmetrical dimers based upon the epoxide domain of the azinomycins. They demonstrated that an optimum linker length appeared to be 4 methylene groups and that the agents can cross-link DNA, and have potent cytotoxic activity, although none of the compounds had significantly greater activity than the non-crosslinking A.

The azinomycins appear to act by disruption of cellular DNA replication by interstrand crosslink formation. Lain et al in Can. J. Biochem. 1997, 55, 630-635 first noted the ability of azinomycin B to form covalent links between complementary strands of DNA. Fujiwara et al in Tetrahedron Lett. 1999,40,315-318 further suggest that the crosslinking occurs via an initial alkylation of the aziridine with the N7 of adenine followed by efficient crosslinking through a second reaction of the N7 of a guanine 2 bases away with the epoxide.

Casely-Hayford et al in Bioorganic and Med. Chem. Letters (2005) 15, 653-656, discuss the design and synthesis of a potentially therapeutically-viable azinomycin analogue B based upon A involving the coupling of a piperidine mustard to the acid chloride of the azinomycin chromophore.

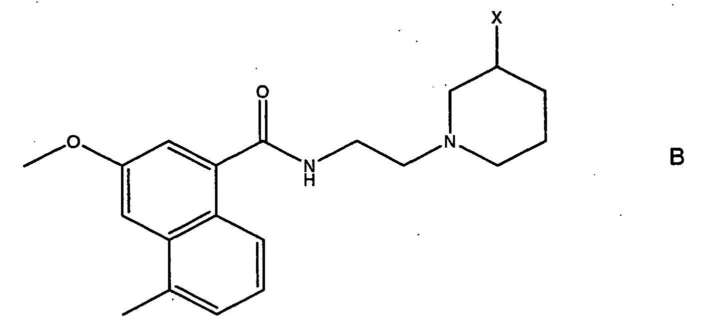

The authors conclude that monoalkylation is sufficient for biological activity and that crosslinking may even be detrimental.

The present invention relates to the first therapeutic use of a range of azinomycin analogues and their synthesis. The compounds incorporated herein are new. The present invention also relates to synthetic precursors of azinomycin analogues which do not have the epoxide or the aziridine ring of the natural products, and which are substantially inactive as DNA alkylating agents themselves.

It has been reported (Murray et al, 1997, Cancer Research, 57, 3026-3031 and WO-A-9712246) that the enzyme CYP1B1, a member of the cytochrone P450 (CYP) family of xenobiotic metabolizing enzymes, is expressed at high frequency in a range of human cancers, including cancers of the breast, colon, lung, oesophagus, skin, lymph node, brain and testes, and that it is not detectable in normal tissue. This led to the conclusion that the expression of cytochrome P450 isoforms in tumour cells provides a molecular target for the development of new anti-tumour drugs that could be selectively activated by the CYP enzymes in tumour cells, although no drug examples were given. A number of other CYP isoforms have been shown to be expressed in various tumors. Many of the CYPs expressed in tumors are mentioned in Patterson, LH et al (1999) Anticancer Drug Des. 14(6) 473-486.

In WO 02/067930A1 Searcey and Patterson describe various benz-indole and benzo-quinoline compounds as CYP-oxidisable prodrugs for tumour treatment. In WO 02/068412A1 they further describe pyrrolo-indole and pyrrolo-quinoline derivatives for use as CYP-oxidizable prodrugs and in WO 02/067937A1 indoline and tetrahydro-quinoline CYP-oxidisable prodrugs are described. All of these compounds are expected to be hydroxylated at the carbon atom to which X is joined by cytochrome P450, in particular CYP1 B1, expressed at high levels in tumors.

The present invention is directed to a new class of prodrugs which are expected to be oxidized in situ by CYP enzymes, in particular enzymes expressed at high levels in tumors. In particular, the prodrugs are believed to be metabolizable by CYP1B1 enzyme. P450 enzymes are involved in Phase I metabolism and are well known to be able to convert an alkene to an epoxide to form an active compound. It is believed that no drugs have previously been activated in this manner. Some of the compounds of the present invention contain nitrogen mustards and may act as alkylating agents.

According to the present invention there is provided novel prodrugs of general formula I or a salt thereof: 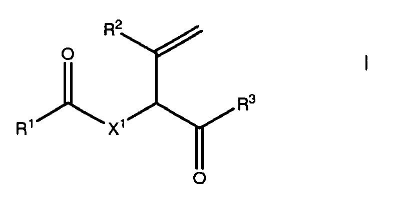 in which X¹ is selected from a group consisting of O, S and NR⁰ in which R⁰ is H or C₁₋₄ alkyl;
R³ is NH₂, NHR⁴, SR⁴, OR⁴, CH₂R⁴ or OH;
R¹ is optionally substituted naphthyl, anthranyl or a group of formula III 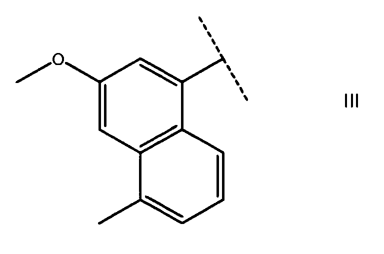 R² is H, optionally substituted C₁₋₄ alkyl, C₁₋₄ alkoxy, optionally substituted phenyl, C₇₋₁₂ aralkyl, optionally substituted heteroaryl or a ligand;
R⁴ is C₁₋₄ alkyl, C₁₋₄ substituted alkyl, C₁₋₄ alkoxy, optionally substituted phenyl, C₇₋₁₂ aralkyl, optionally substituted heteroaryl, CₙH₂ₙNR⁵R⁶ or a ligand;
in which at least one of R⁵ and R⁶ is (CH₂)₂A¹ or together with the nitrogen to which they are attached form a ring of formula II 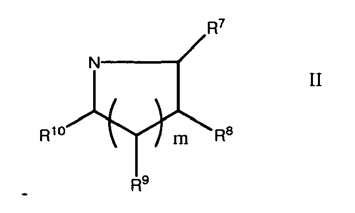 in which at least one of R⁷, R⁸ and R⁹ is selected from A¹ and A¹ substituted C₁₋₄ alkyl, and any others are H or C₁₋₄ alkyl; R¹⁰ is selected from H, C₁₋₄ alkyl, A¹ and A¹ substituted C₁₋₄ alkyl;
A¹ is a leaving group or a halogen atom;
m is 1-4;
n is 1-7;
wherein the susbtituent groups are selected from C₁₋₄ alkyl, hydroxyl, amino, alkyl amino, halo and aziridine, and the ligand is an oligopeptide, biotin, avidin, streptavidin, a polymeric group, an oligonucleotide or a protein.

Suitable examples of halogen atoms are fluorine, chlorine, bromine and iodine, preferably chlorine. Suitable examples of leaving groups are alkyl or aryl sulphonates, carboxylates, alkyloxy, acyloxy and aryloxy groups.

In the present invention the term ligand includes a group having specific targeting characteristics, useful for instance in antibody or gene-directed enzyme prodrug-type environments. A ligand may be an oligopeptide, biotin, avidin or streptavidin, a polymeric group, an oligonucleotide or a protein. Preferably it has specific binding characteristics and is preferably an antibody or fragment, an antigen, a sense or anti-sense oligonucleotide, or one of avidin, streptavidin and biotin, that is one component of a specific binding pair. Alternatively, it may be a group designed for passive targeting, such as a polymeric group, or a group designed to prolong the stability or reduce immunogenicity such as a hydrophilic group. US-A-5843937 discloses suitable ligands for conjugating to these types of actives and methods for carrying out the conjugation.

In these compounds, the group R¹ is chosen so that it facilitates the intercalation of the compound into DNA. For optimized DNA binding ability, the group R¹ is selected from the group consisting of optionally substituted naphthyl and anthranyl. When R¹ is optionally substituted naphthyl, excellent intercalation is observed.

A preferred group for R¹ is III

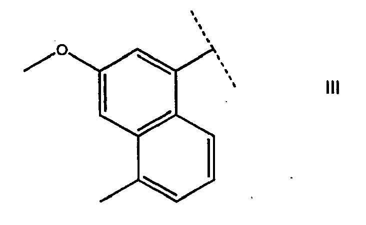

In the compounds of the present invention X¹ is preferably oxygen, although sulphur and nitrogen analogues have been generated and have useful properties.

In one preferred embodiment X¹ is O, R² is CH₃ and R³ is NH₂.

Two examples of such a class of compounds are

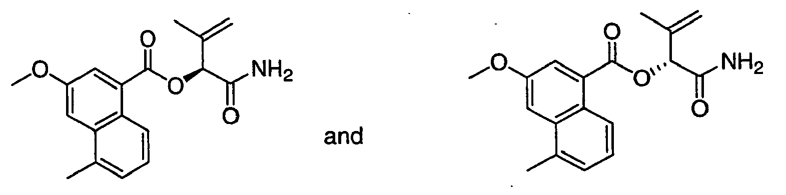

Amide analogues of these compounds have been generated and represent a further embodiment of the present invention. The following allylglycine derivative exemplifies this embodiment:

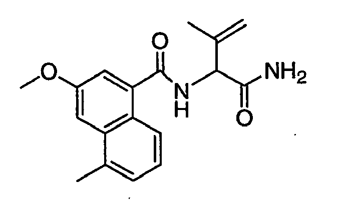

The compounds of the present invention may be present as racemic mixtures or as isolated R or S enantiomers. It is often found that one enantiomer shows more biological activity than another and is therefore preferred.

These compounds are converted into epoxides in vivo by a CYP-mediated biooxidative process. This is shown in the diagram below.

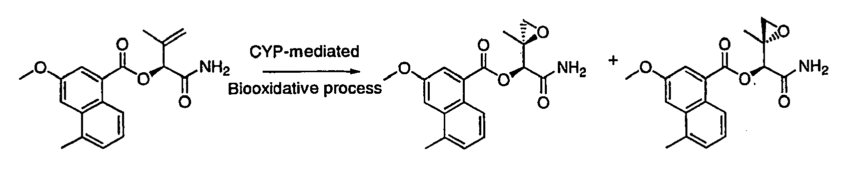

The activated products, the epoxides, of this preferred class of compounds of the invention monoalkylate DNA through the epoxide at the N7 of guanine in the major groove. Nitrogen mustards, that alkylate DNA through the mustard moiety but have the potential to become crosslinking agents via formation of an epoxy group form preferred embodiments of the present invention. Although nitrogen mustards themselves have potent anti-tumour activity, it is believed that conversion to a crosslinking agent through CYP-mediated bioxidation could lead to enhancement of activity or a change in the relative spectrum of activity of a compound.

Accordingly, a second class of preferred compounds of the present invention of general formula I have R³=NHR⁴, wherein R⁴ is a group of formula CₙH₂ₙNR⁵R⁶ as defined above. R⁵ and R⁶ may be joined to form a ring of general formula II. The compounds of the present invention may be pyrrolidine derivatives, that is in which m=1. Another class of compounds of the invention are piperidine derivatives, in which m=2.

In a preferred class of such compounds of the present invention
i) R⁷ is CH₂A¹ and R⁸ is H; or
ii) R⁷ is H and R⁸ is A¹.

In this embodiment R¹⁰ is H or is the same group as R⁷ and the or each R⁹ is H or the same group as R⁸. Such compounds have been shown to cross-link duplex DNA at concentrations similar to those given for the natural products Azinomycin A and B or close analogues. However, the compounds of the present invention are more stable and therapeutically robust, showing greater potential as anti-tumour agents.

Compounds in which the groups R⁷ and R⁸ are not one of the definitions mentioned above in connection with alkylating agents, may nevertheless bind to DNA and cause cytotoxicity.

A preferred structure of group CₙH₂ₙNR⁵R⁶, wherein n=2 is shown below.

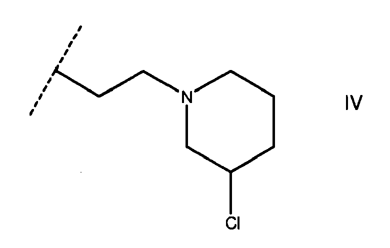

A specific example of this second class of compounds of formula I which contains a nitrogen mustard and may be biooxidatively activated is

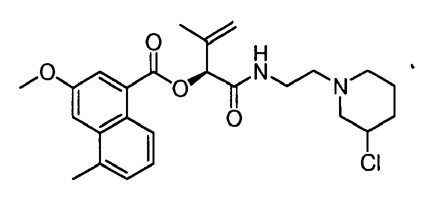

One particular isoform of the cytochrome P450 family of enzymes, CYP1B1, is thought to be tumour specific. This provides for a self-targeting drug delivery system in which a non-toxic (or negligibly cytotoxic) compound can be administered to a patient, for example, in a systematic manner, the compound then being activated at the site of the tumour cells to form a highly cytotoxic compound which acts to kill the tumour cells.

According to the present invention there is also provided a synthetic method in which a compound of formula V 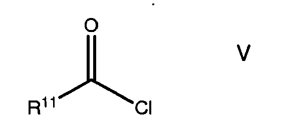 in which R¹¹ is selected from a group consisting of optionally substituted naphthyl, anthranyl and a group of formula III; is reacted with a compound of formula VI 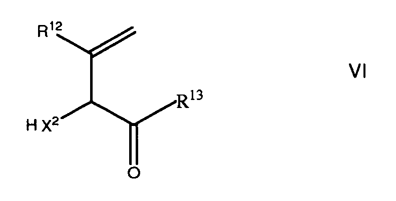 in which R¹² is H, optionally substituted C₁₋₄ alkyl, C₁₋₄ alkoxy, optionally substituted phenyl, C₇₋₁₂ aralkyl, optionally substituted heteroaryl or a ligand;
X² is O, NH or S;
R¹³ is OH, Cl, C₁₋₄ alkoxy or OPG wherein PG is a protecting group;
such that Cl in V is replaced in a nucleophilic substitution reaction by a group of formula VII 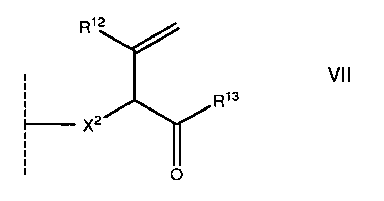 wherein the ligand is an oligopeptide, biotin, avidin, streptavidin, a polymeric group, an oligonucleotide or a protein.

The group R¹³ preferably incorporates a protecting group to ensure that the X² substituent acts as the nucleophilic end of the molecule. Suitable protecting groups for alcohols include benzyl ether, trialkyl silyl (e.g. TBDMS) and tetrahydropyranyl (THP). Of these, benzyl ether is preferred.

Once the coupling is complete the protecting group may be removed by a deprotection reaction. In a preferred embodiment, the protecting group is benzyl ether and this may be removed using H₂ over a Pd/C catalyst or by using HBr reagent to yield a carboxylic acid.

The carboxylic acid may then be reacted with a suitable nucleophile, HR¹⁴, wherein R¹⁴ is selected from the group consisting of NH₂, NHR¹⁵, SR¹⁵ and OR¹⁵ to give a compound of formula VIII 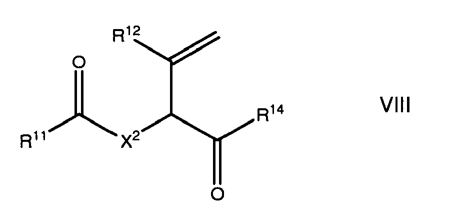 wherein R¹⁵ is selected from the group consisting of C₁₋₄ alkyl, C₁₋₄ substituted alkyl, C₁₋₄ alkoxy, optionally substituted phenyl, C₇₋₁₂ aralkyl, optionally substituted heteroaryl, CₚH₂ₚNR¹⁶R¹⁷ and a ligand;
in which at least one of R¹⁶ and R¹⁷ is (CH₂)₂A² or together with the nitrogen to which they are attached form a ring of formula IX 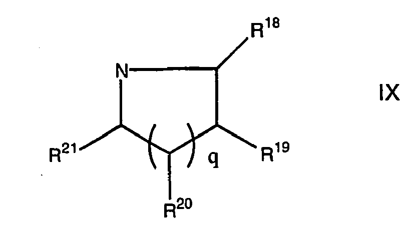 in which at least one of R¹⁸, R¹⁹ and R²⁰ is selected from A² and A² substituted C₁₋₄ alkyl, and any others are H or C₁₋₄ alkyl;
R²¹ is selected from H, C₁₋₄ alkyl, A² and A² substituted alkyl;
A² is a leaving group, hydroxyl, protected hydroxyl or a halogen atom;
q is 1-4;
p is 1-7;
wherein the substituent groups are selected from C₁₋₄ alkyl, hydroxyl, amino, alkyl amino, halo and aziridine.

The product of the above synthetic method may be oxidized at the alkene to which R¹² is attached to form the corresponding active compound. Suitable reagents for carrying out this conversion include Dimethyl dioxirane (DMDO), hydrogen peroxide, the peroxycarboxylic acids and the peroxy-acids, for example meta-chloroperbenzoic acid.

In a synthesis of compounds of the present invention which contain the ring of formula IX, or a CₚH₂ₚNR¹⁶R¹⁷ group, the groups R¹⁶-R²¹ may be the same as in the desired end product of general formula R⁵-R¹⁰. Alternatively, these groups may be precursors for the desired end groups and may be replaced in a subsequent reaction step or steps to generate the desired substituent. Examples of subsequent reaction steps would be halogenating steps carried out on a hydroxyl, or protected hydroxyl after deprotection, group. In such processes a group A² which is hydroxyl or a protected hydroxyl group, is reacted with a halogenating agent, such as a chlorinating agent, optionally after deprotection, to replace the or each A² by a halogen atom. Preferably this halogen atom is chlorine.

In the synthesis, R¹¹ is optionally substituted naphthyl, anthranyl or preferably a group of formula III.

X² is preferably oxygen, R¹² is preferably methyl and R¹⁴ is preferably NH₂ or CₚH₂ₚNR¹⁶R¹⁷, wherein R¹⁶ and R¹⁷, together with the nitrogen to which they are attached form a ring of formula IX.

Intermediates for the synthesis of the compounds of general formula I of the present invention are believed to be new compounds and may be represented by the general formula X 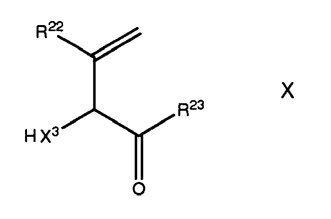 in which X³ is selected from the group consisting of O, NH and S;
R²² is H, C₁₋₄ alkyl, C₁₋₄ alkoxy, optionally substituted phenyl, C₇₋₁₂ aralkyl, optionally substituted heteroaryl or a ligand;
R²³ is a ligand or NHR²⁴ wherein R²⁴ is CᵣH₂ᵣNR²⁵R²⁶ or a ligand;
R²⁵ and R²⁶ are (CH₂)₂A³ or both together with the nitrogen to which they are attached, form a ring of formula XI 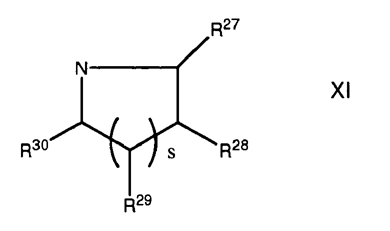 in which at least one of R²⁷, R²⁸ and R²⁹ is selected from A³ and A³ substituted C₁₋₄ alkyl and any others are H or C₁₋₄ alkyl, R³⁰ is selected from H, C₁₋₄ alkyl, A³ and A³ substituted C₁₋₄ alkyl;
A³ is a leaving group, OH, protected hydroxyl or a halogen atom;
s is 1-4;
r is 1-7.

In the intermediates of the present invention, R²² is preferably CH₃. X³ is preferably O and as in the compounds of the present invention of general formula I, R²⁵ and R²⁶ preferably form a ring together with the nitrogen to which they are attached, to give a nitrogen mustard.

The groups R²⁷-R³⁰ may be the same or different to the groups R⁷-R¹⁰ in compound II. If different, the groups R²⁷-R³⁰ may be converted to corresponding R⁷-R¹⁰ in a subsequent reaction step.

A specific example of novel intermediate is

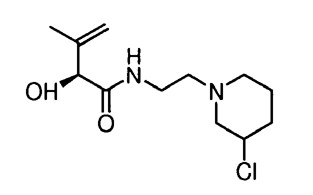

The present invention provides novel prodrugs which have a DNA - intercalating group R¹ and a nitrogen mustard which alkylates DNA.

Suitable examples of halogen atoms are fluorine, chlorine, bromine and iodine, preferably chlorine. Suitable examples of leaving groups as A⁴ are carboxylates, alkyl sulphonates, aryl sulphonates, alkyloxy, acyloxy and aryloxy groups.

The compounds of the present invention may be present as racemic mixtures or as isolated R- or S- enantiomers. It is often found that one enantiomer shows more biological activity and is therefore preferred.

Suitable protecting groups for alcohols include benzyl ether, trialkyl silyl (e.g. TBDMS) and tetrahydropyranyl (THP). Of these, benzyl ether is preferred.

The compounds of the present invention of general formula I may be useful in a method of treatment of an animal by therapy. In particular, the cytotoxic properties of the compound itself or the activated form, as the case may be, may be useful in anti-tumour treatment. The invention further provides the use of these compounds in the manufacture of compositions for use in a method of treatment of an animal. The compounds may be incorporated into a pharmaceutical composition together with a pharmaceutically acceptable excipient.

Pharmaceutical compositions may be suitable for intramuscular, intraperitoneal, intrapulmonary, oral or, most preferably, intravenous administration. The compositions may contain suitable matrixes, for example for controlled or delayed release. The compositions may be in the form of solutions, solids, for instance powders, tablets or implants, and may comprise the compound of the formula I in solid or dissolved form. The compound may be incorporated in a particulate drug delivery system, for instance in a liquid formulation. Specific examples of suitable excipients include lactose, sucrose, mannitol, and sorbitol; starch from corn, wheat, rice, potato, or other plants; cellulose, such as methyl cellulose, hydroxypropylmethyl-cellulose, or sodium carboxymethylcellulose; gums, including araboc and tragacanth; and proteins, such as gelatin and collagen. If desired, disintegrating or solubilising agents may be added, such as the cross-linked polyvinyl pyrrolidone, agar, and alginic acid or a salt thereof, such as sodium alginate. Solid compositions may take the form of powders and gels but are more conveniently of a formed type, for example as tablets, cachets or capsules (including spansules). Alternative, more specialised types of formulation include liposomes, nanosomes and nanoparticles.

The animal which is treated is generally human, although the compounds may also have veterinary use. The indication treated is generally cancer, including adenocarcinoma, leukemia, lymphoma, melanoma, myeloma, sarcoma, teracarinoma, and, in particular, cancers of the adrenal gland, bladder, bone, bone marrow, brain, breast, cervix, gall bladder, ganglia, gastrointestinal tract, heart, liver, kidney, lung, muscle, ovary, pancreas, parathyroid, penis, prostate, salivary glands, skin, spleen , testes, thymus, thyroid and uterus. The tumour may, for instance, be defined as a tumour expressing high levels of CYP1B1.

The oxidised forms of the prodrugs of the first aspect of the present invention and the mustard compounds of the second aspect of the invention alkylate DNA and cause cytotoxicity. As such, they are potent cytotoxic agents whose exact biological mechanism of action is unknown but involves the disruption of template and other functions of DNA. General inhibition of template function of DNA will affect all dividing cells in the body and lead to unacceptable side effects in a therapeutic setting. However, the targeted production of the epoxide forms only in tumour cells that over express particular isoforms of cytochrome P450 will lead to a specific cytotoxic effect only in those cells.

### Brief Description of the Figures

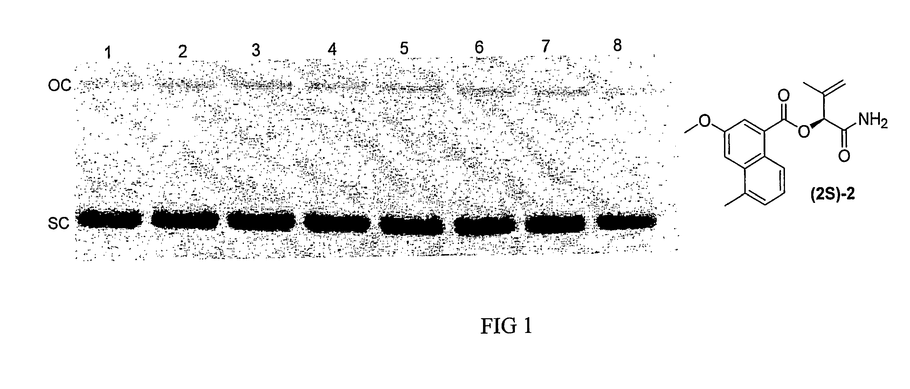

FIG. 1: Effect of **2** on the electrophoretic mobility of F174 plasmid DNA.
FIG. 2:
   (a) cytotoxicity of **2** on CHO cells (with or without CYP3A4).
   (b) cytotoxicity of mach 361/1 ((25,35)-(1)) on CHO cells (with or without CYP3A4).
FIG. 3:
   (a): Effect of **20** on DNA crosslinking after 1 h incubation with pUC18 plasmid DNA.
   (b) The percentage crosslinked (double stranded) DNA.
FIG. 4: As for FIG. 3 but after 2h incubation.
FIG. 5: As for FIG. 3 but after 3h incubation.

The following examples illustrate the invention:

### Example 1

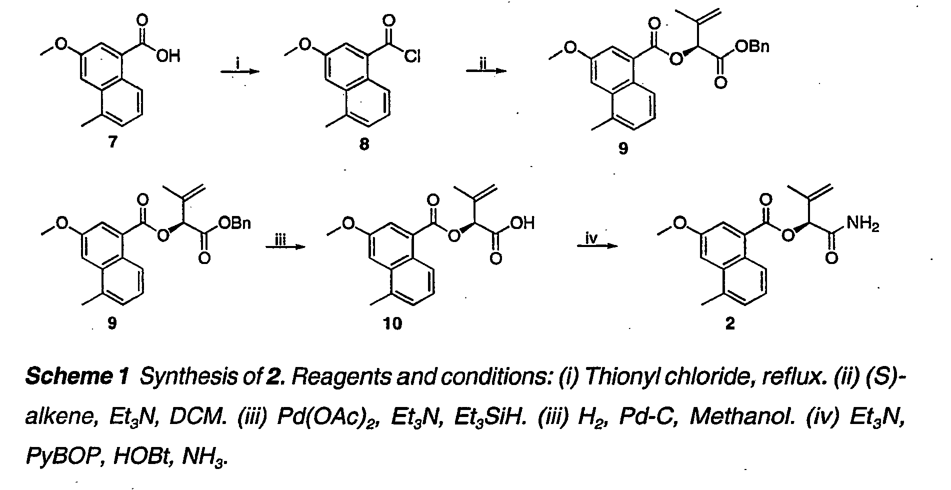

The novel alkene amides of general formula I were prepared from the carboxylic acid **7** in four steps. The acid chloride **8** was coupled to the benzyl hydroxybutenoate by dropwise addition to a stirred solution of alcohol together with Et₃N in dry CH₂Cl₂ under a nitrogen atmosphere at 0 °C. After 4 h the reaction was quenched with H₂O extracted with CH₂Cl₂ and purified to give **9** in 65 % yield. Proton NMR analysis confirmed the structure and showed the alkenyl protons as multiplets at 5.33 and 5.18 ppm. The benzyl CH₂ protons also appeared as a multiplet at 5.31 ppm, the H-2 proton was detected at 5.77 ppm and the methyl hydrogens had values of 2.68 ppm for the aromatic methyl and 3.96 ppm for the methoxy methyl.

The benzyl group was selectively deprotected using catalytic Pd(OAc)₂. A solution of the Pd(OAc)₂, Et₃N and Et₃SiH in dry CH₂Cl₂ was stirred at RT under N₂ for 15 min. A solution of the ester **9** in dry CH₂Cl₂ was then added dropwise. The mixture was stirred at RT overnight before quenching the reaction by the addition of NH₄Cl. After extraction with Et₂O the alkenyl carboxylic acid was recovered in 90% yield. This acid was then treated with 35 % NH₃, Et₃N, HOBt and PyBOP to give the amide **2** in 66 % yield. NMR analysis showed the NH₂ protons as broad singlets at 6.13 and 5.65 ppm whereas the H-2 proton appeared at 5.87 ppm. The alkene methylene protons were identified as two multiplets at 5.36 and 5.21 ppm, and the methyl groups as singlets at 3.95 (OCH₃), 2.52 (Ar-CH₃) and 1.96 ppm (CH₃). The aromatic protons on the naphthalene chromophore were at 8.65 (1 H), 7.90 (1 H), 7.50 (1 H) and 7.36 ppm (2H). The stereoisomer, compound (*R*)-**2** was synthesised using the same route but employing (*R*)-hydroxy butenoate.

### Example 2

### Preliminary Biological investigations of potential bio-oxidative prodrugs

Initial cytotoxicity studies were in the performed U2-OS and HoeR cell lines. The U2-OS is a human osteosarcoma cell line and HoeR is a DNA minor groove binder-resistant variant of this. Both are available from the American Type Culture Collection (ATCC), Dr. Raymon H.10801 University Boulevard, Manassas, VA, 20110-2209, USA. The studies revealed that the alkene amide analogues **2** and (*R*)-**2** were not cytotoxic compounds whereas their epoxide counterparts (2*S*, 3*S*)-**1**, (2*S*, 3*R*)-**1**, (2*R*, 3*R*)-**1**, (2*R*, 3*S*)-**1** demonstrated good activity in these cell lines (Table 1).

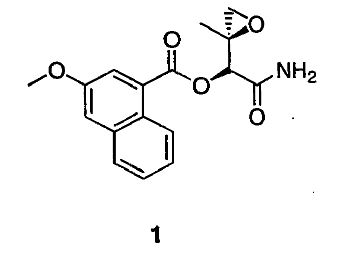

**Table 1 IC₅₀ values in U2-OS and HoeR. U2 OS is a human osteosarcoma cell line, HoeR is a Hoechst415 resistant version of U2-OS.**

| Panel/ Cell line | IC₅₀ (nM) of compound | | | | | |
|---|---|---|---|---|---|---|
| | (2*S*, 3*S*)-**1** | (2*S*, 3*R*)-**1** | (2*R*, 3*R*)-**1** | (2*R*, 3*S*)-**1** | **(2S)-2** | **(2R)-2** |
| U2-OS | 15 | 120 | 40 | 40 | >10 000 | >10 000 |
| HoeR | 14 | 121 | 40 | 45 | >10 000 | >10 000 |

Figure 1 shows the effect of **2** on the electrophoretic mobility of F174 plasmid DNA. Lane 1 contains DNA only, lanes 2-8 have 10⁻³, 10⁻², 10⁻¹, 1,10, 20 and 30 drug/bp ratio respectively. The DNA concentration was 3.8 µm and SC stands for supercoiled DNA and OC for Open Circular DNA.

### Example 3

### Preliminary Metabolism Studies

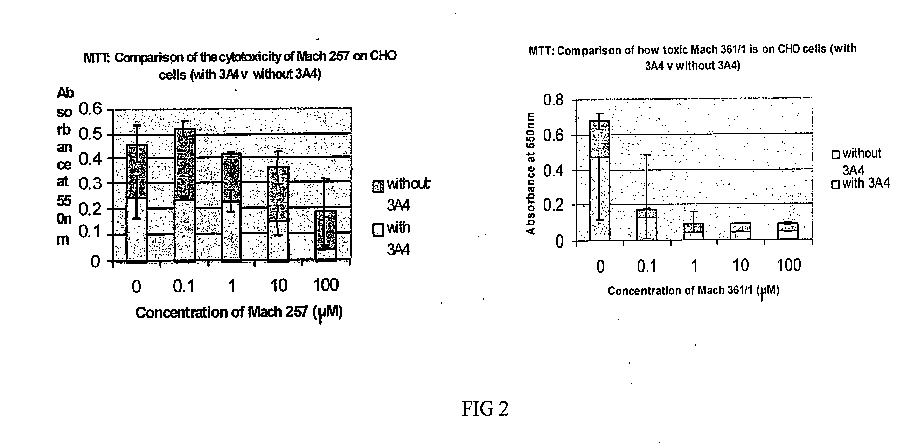

Table 1 shows that **2** lacks cytotoxic activity in U2-OS and HoeR cell lines in vitro at concentrations as high as 10 µM. Further studies of **2** in wild type CHO cells and CHO cells that have been transfected with CYP3A4 revealed that the prodrug **2** appears more cytotoxic in CYP3A4 CHO cells compared to wild type (absent in CYP3A4) Figure 2(a) shows the cytotoxicity of **2** on CHO cells, with or without CYP3A4.

Figure 2(b) similarly shows the cytotoxicity of (25, 35)**-1.** By comparison the epoxide (active) compound has high cytotoxicity in either cell line. This is an initial indicator that shows that the alkene functionality can indeed be metabolised by cytochrome P-450 enzymes to a compound, which is more cytotoxic than the parent alkene precursor.

### Reference Example 4

The synthesis of a compound of general formula XII was carried out according to schemes 2 and 3.

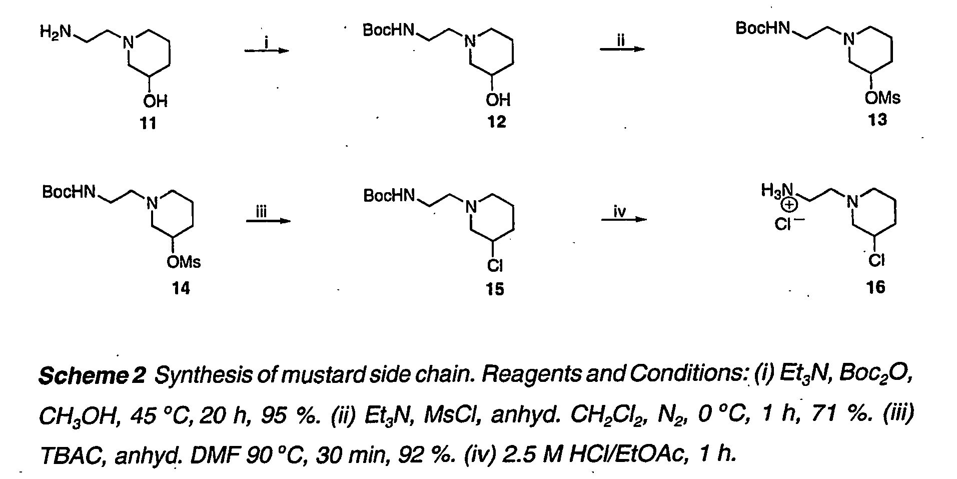

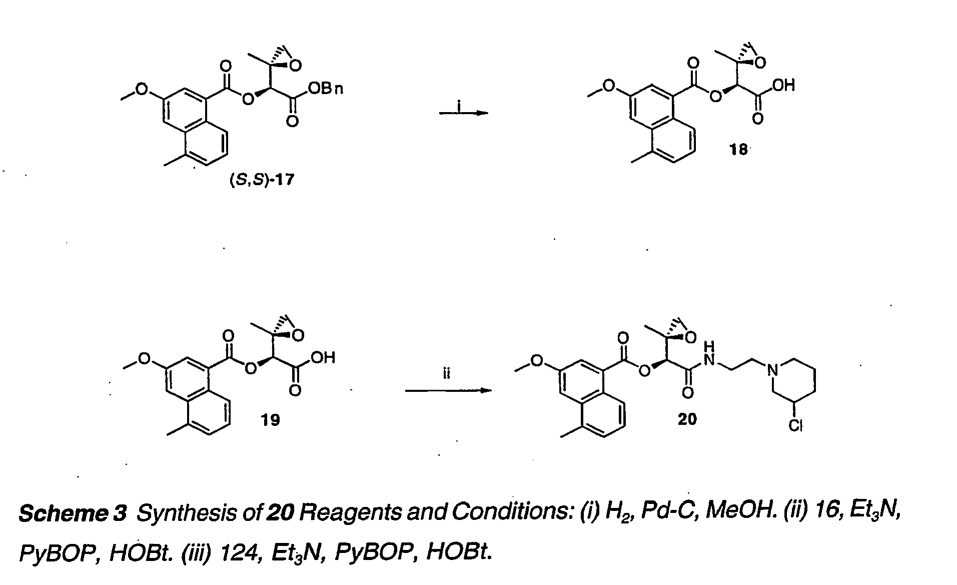

The 2-chloropiperidine **16** was synthesised from 1-(2-aminoethyl)-piperidin-3-ol **11** following Boc-protection of the primary amine. This was achieved by stirring the diamino alcohol **11** in CH₃OH for 5 min after which Boc₂O (dissolved in CH₃OH) was added dropwise over 20 min and the reaction mixture stirred at 45°C for 20 h. It was concentrated in vacuo, dissolved in EtOAc and washed with H₂O to afford **12** as a straw coloured oil in 95 % yield.

The Boc-protected amine **12** was then converted to the mesylate **13** by stirring in anhyd. CH₂Cl₂, with Et₃N and adding MsCl dropwise at 0 °C. After 1 h the reaction was quenched with ice cold NaHCO₃ in brine and extracted with cold CH₂Cl₂ to give **14** the precursor to the Boc protected 2-chloropiperidine derivative in 71 % yield. The mesylate was immediately transformed into the Boc-protected mustard **15** by heating in anhyd. DMF to 90°C in the presence of TBAC for 30 min after which the DMF was removed in vacuo and the reaction residue redissolved in CH₂Cl₂ and washed with cold NaHCO₃ to give the Boc-protected mustard **15** in 92 % yield. Prior to coupling to the carboxylic acid functionality of the left hand portion of the azinomycins, the Boc-protected amine was deprotected by stirring in dry 2.5 M HCl in EtOAc for an hour. EtOAc was then removed by evaporation to give the chloride salt of the amine.

The benzylester (*S*, *S*)-**17** was synthesised using a stereoselective method as described in Bryant et al in Synlett. 1996, 10, 973. **17** is converted to the free epoxy carboxylic acid in Scheme 3, step (i), by hydrogenolysis using Pd-C in CH₃OH under hydrogen atmosphere.

To prepare **20,** the freshly prepared epoxy carboxylic acid was dissolved in dry DMF, stirred at 0 °C and was successively treated with **16,** Et₃N and PyBOP. The reaction mixture was then warmed to RT and stirred for 18h after which toluene was added and the resulting solution successively washed with NaHCO₃ and brine. Column chromatography (10-20 % CH₃OH/CH₂Cl₂) provided **20** in 67% yield.

### Reference Example 5 DNA Crosslinking

Plasmid DNA pUC 18 was linearised by digestion with Hind III. The linear DNA was then dephosphorylated with BAP and ³²P-radiolabelled on the 5'-end. The DNA was then purified by EtOH precipitation to remove unincorporated γ-³²P ATP and the DNA resuspended in sterile double distilled H₂O. To each reaction sample was added ³²P-end labelled DNA and drug at the appropriate concentration. Following incubation at 37°C for the required time, the reaction was terminated by the addition of Stop Solution Buffer. The DNA-drug adduct was EtOH precipitated and dried by lyophilisation. Each dried DNA sample including an untreated DNA single strand as a control was denatured by resuspending in alkali denaturing buffer. The double stranded control DNA was then dissolved in sucrose loading buffer and the samples loaded and electrophoresed on a 20 cm long 0.8 % horizontal agarose gel submerged in 1 x TAE buffer at 40 V for 16h. Gels were then dried and autoradiographed

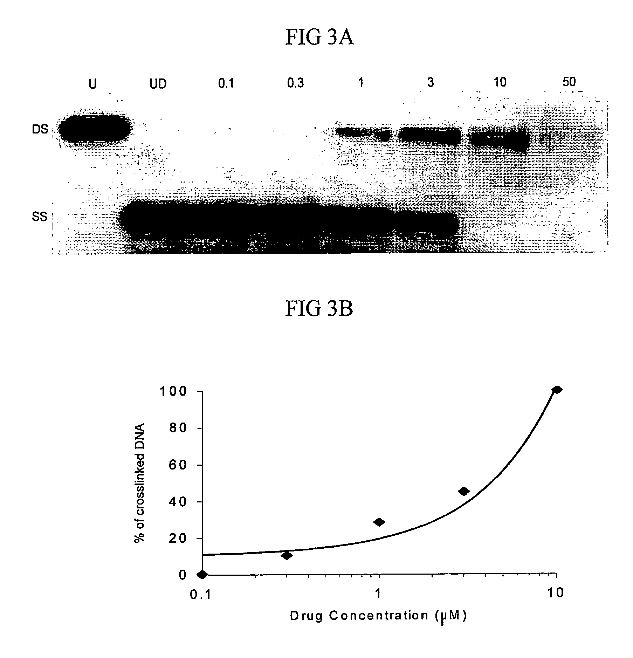

Compound **20** which consists of both the epoxide and mustard functionality was tested at concentrations between 0.1 and 50 µM at 1h, 2h and 3h intervals. Figure 3 displays an autoradiograph and a concentration-response curve showing that **20** can imitate the natural product Azinomycin A and crosslinks linear double stranded plasmid pUC18 DNA after one hour incubation. Crosslink formation starts at concentrations as low as 0.1 µM and reaches 100 % crosslinking at -10 µM. After incubation for an hour the CR₅₀ (concentration at which 50% of the duplex is crosslinked) was determined to be 3.1 µM. The percentage of crosslinked DNA was determined from autoradiograph densitometry.

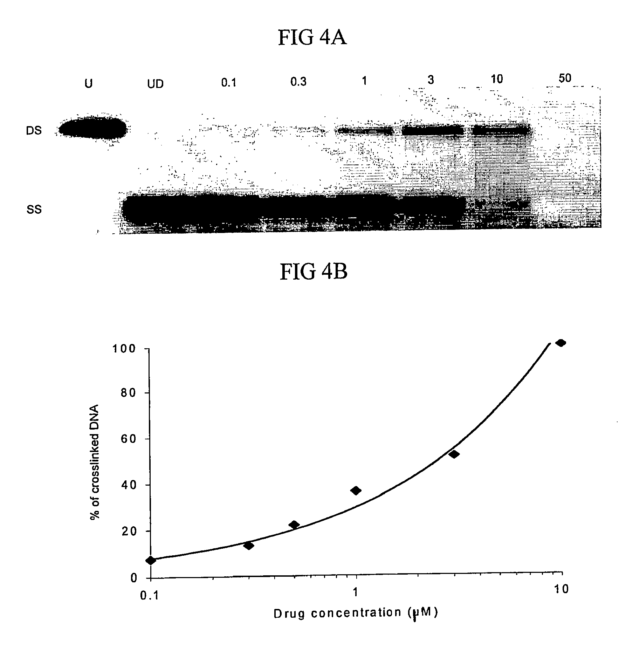

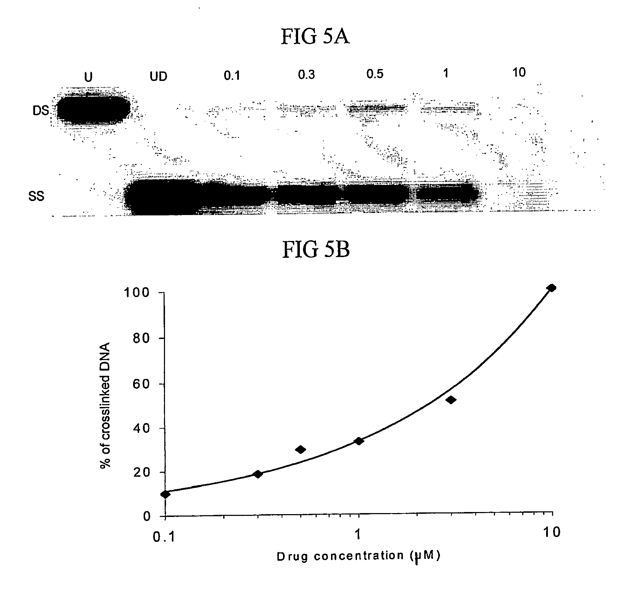

Crosslink formation progressed steadily over time and after 2 h the CR₅₀ was reduced from 3.1µM to 2.7 µM (Figure 4). After 3 h the CR₅₀ was 2.2 µM (Figure 5).

In Figures 3-5 DS stands for double stranded DNA, SS stands for single stranded DNA, U for untreated nondenatured DNA and UD for untreated denatured DNA.

### Reference Example 6 Antitumour Activity

Examples 6 and 7 make use of the NCI 60 cell panel. This is an in vitro cell line screening project providing direct support to the National Cancer Institute's USA Developmental Therapeutic Programme for anti cancer discovery. The methodolgy for the cell line's operation is described by Boyd et al in Drug Development Research 1995, 34, 91-109.

The antitumour activity in the NCI 60 cell line panel shows the compound to have low micromolar to high nanomolar activity. Table 2 shows the anti-tumour activity (GI₅₀, µm) of compound **20,** where the GI₅₀ value is the concentration which results in growth inhibition of 50%.

**Table 2**

| Cell line | Compound **20** |
|---|---|
| ***Leukemia*** | |
| CCRF-CEM | 3.26 |
| HL-60(TB) | 6.98 |
| K562 | 8.33 |
| MOLT-4 | 3 |
| RPMI-8226 | 4.18 |
| SR | 2.02 |

| ***NSCLC*** | |
|---|---|
| A549/ATCC | 31.1 |
| EKVX | 17.5 |
| HOP-62 | 100 |
| HOP-92 | 13.8 |
| NCI-H226 | |
| NCI-H23 | |
| NCI-H322M | 17.9 |
| NCI-H460 | 13 |
| NCI-H522 | 7.77 |

| ***COLON*** | |
|---|---|
| COLO 205 | 13.8 |
| HCC2998 | 8.25 |
| HCT-116 | 100 |
| HCT-15 | |
| HT29 | 11 |
| KM12 | 14.5 |
| SW-620 | 5.26 |

| ***CNS*** | |
|---|---|
| SF-268 | 16.6 |
| SF-295 | 13.1 |
| SF-539 | 6.27 |
| SNB-19 | 14.3 |
| SNB-75 | |
| U251 | 7.82 |

| ***MELAN*** | |
|---|---|
| LOX IMVI | 3.62 |
| MALME-3M | 14.8 |
| M14 | 100 |
| SK-MEL-2 | 18.6 |
| SK-MEL-28 | 11.6 |
| SK-MEL-5 | 1.56 |
| UACC-257 | |
| UCC-62 | 14.7 |

| ***OVAR*** | |
|---|---|
| IGROV1 | 12.1 |
| OVCAR-3 | 18.6 |
| OVCAR-4 | 21.5 |
| OVCAR-5 | 16.1 |
| OVCAR-8 | 44.5 |
| SKOV-3 | 19.7 |

| ***RENAL*** | |
|---|---|
| 786-0 | 100 |
| A498 | 17.7 |
| ACHN | 1.17 |
| CAKI-1 | 10.3 |
| RXF 393 | 17.5 |
| SN12C | 6.76 |
| TK10 | 23.4 |

| ***PROST*** | |
|---|---|
| PC-3 | 11.2 |
| DU-145 | 4.49 |

| ***BREAST*** | |
|---|---|
| MCF7 | 14.7 |
| NCI/ADRRES | 11.1 |
| MDA-MB- | |
| 231/ATCC | 14 |
| HS 578T | 17.5 |
| 435 | 14.3 |
| BT-549 | 12.5 |
| T-47D | 23 |
| MGMID | 12.3 |

### Example 7

A similar method to that used in Example 4 (Schemes 2 and 3) was used to synthesise the alkylating analogue using the mustard **16** and alkene carboxylic acid **10** to give **21** (59% yield).

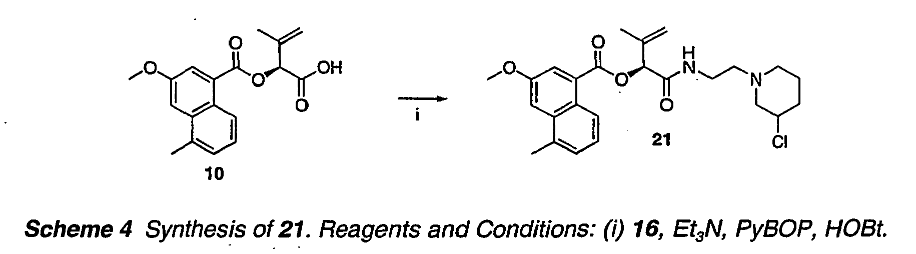

### Antitumour Activity

Table 3 shows the anti-tumour activity (GI₅₀, µM) of **21.**

**Table 3**

| Cell line | Compound **21** |
|---|---|
| ***Leukemia*** | |
| CCRF-CEM | 3.24 |
| HL-60(TB) | 12.1 |
| K562 | 5.16 |
| MOLT-4 | 3.3 |
| RPMI-8226 | 3.43 |
| SR | 2.34 |

| ***NSCLC*** | |
|---|---|
| A549/ATCC | 8.26 |
| EKVX | 11.4 |
| HOP-62 | 3.45 |
| HOP-92 | 4.12 |
| NCI-H226 | |
| NCI-H23 | |
| NCI-H322M | 8.07 |
| NCI-H460 | 6.26 |
| NCI-H522 | 5.1 |

| ***COLON*** | |
|---|---|
| COLO 205 | 6.08 |
| HCC2998 | 4.27 |
| HCT-116 | 12.8 |
| HCT-15 | 0.049 |
| HT29 | 7.26 |
| KM12 | 9.3 |
| SW-620 | 2.76 |

| ***CNS*** | |
|---|---|
| SF-268 | 11.9 |
| SF-295 | 4.35 |
| SF-539 | 0.45 |
| SNB-19 | 5.63 |
| SNB-75 | 1.2 |
| U251 | 2.63 |

| ***MELAN*** | |
|---|---|
| LOX IMVI | 2.84 |
| MALME-3M | 7.51 |
| M14 | 1.75 |
| SK-MEL-2 | 7.81 |
| SK-MEL-28 | 8.48 |
| SK-MEL-5 | 1.99 |
| UACC-257 | |
| UCC-62 | 4.41 |

| ***OVAR*** | |
|---|---|
| IGROV1 | 4.21 |
| OVCAR-3 | 10.2 |
| OVCAR-4 | 20.9 |
| OVCAR-5 | 11.1 |
| OVCAR-8 | 32.9 |
| SKOV-3 | 17.2 |

| ***RENAL*** | |
|---|---|
| 786-0 | 4.48 |
| A498 | 0.19 |
| ACHN | 1.78 |
| CAKI-1 | 5.66 |
| RXF 393 | 19.1 |
| SN12C | 3.76 |
| TK10 | 12.1 |

| ***PROST*** | |
|---|---|
| PC-3 | 7.96 |
| DU-145 | 5.15 |

| ***BREAST*** | |
|---|---|
| MCF7 | 4.62 |
| NCI/ADRRES | 12.8 |
| MDA-MB- | |
| 231/ATCC | 11 |
| HS 578T | 3.69 |
| 435 | 7.62 |
| BT-549 | 4.55 |
| T-47D | 10.8 |
| MGMID | 5.12 |

The results from examples 6 and 7 show that compounds 20 and 21 have significant cytotoxicity in the low micromolar range across a wide range of human tumour cell lines.

### Example 8

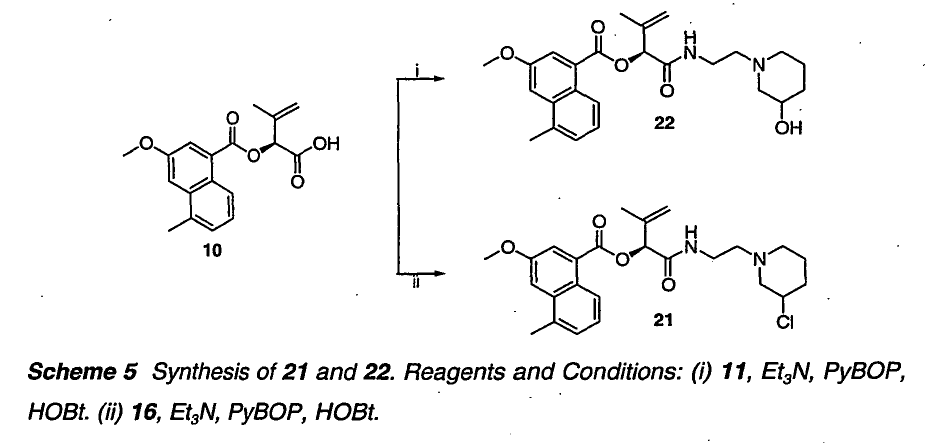

To synthesise the non-alkylating analogue **22,** compound **10** the carboxylic acid intermediate was coupled with hydroxypiperidine **11** in 66% yield using PyBOP methodology. **21** was synthesised as described in Example 7.

## Claims

1. A compound of formula I or a salt thereof 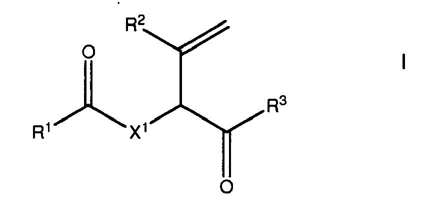 in which X¹ is selected from a group consisting of O, S and NR⁰ in which R⁰ is H or C₁₋₄ alkyl;
R³ is NH₂, NHR⁴, SR⁴, OR⁴, CH₂R⁴ or OH;
R¹ is optionally substituted naphthyl, anthranyl, or a group of formula III 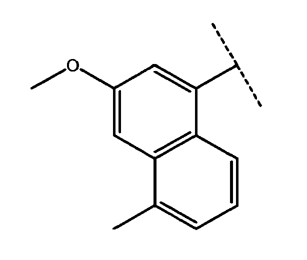 R² is H, optionally substituted C₁₋₄ alkyl, C₁₋₄ alkoxy, optionally substituted phenyl, C₇₋₁₂ aralkyl, optionally substituted heteroaryl or a ligand;
R⁴ is C₁₋₄ alkyl, C₁₋₄ substituted alkyl, C₁₋₄ alkoxy, optionally substituted phenyl, C₇₋₁₂ aralkyl, optionally substituted heteroaryl, CₙH₂ₙNR⁵R⁶ or a ligand;
in which at least one of R⁵ and R⁶ is (CH₂)₂A¹ or together with the nitrogen to which they are attached form a ring of formula II 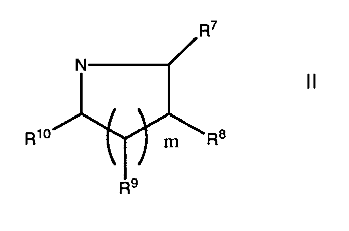 in which at least one of R⁷, R⁸ and R⁹ is selected from A¹ and A¹ substituted C₁₋₄ alkyl, and any others are H or C₁₋₄ alkyl; R¹⁰ is selected from H, C₁₋₄ alkyl, A¹ and A¹ substituted C₁₋₄ alkyl;
A¹ is a leaving group or a halogen atom;
m is 1-4;
n is 1-7;
wherein the substituent groups are selected from C₁₋₄ alkyl, hydroxyl, amino, alkyl amino, halo and aziridine and the ligand is an oligopeptide, biotin, avidin, streptavidin, a polymeric group, an oligonucleotide or a protein.

2. A compound according to claim 1 in which X¹ is O.

3. A compound according to claim 1 or claim 2 in which R² is CH₃.

4. A compound according to any of claims 1-3 in which R³ is NHR⁴.

5. A compound according to claim 4 in which R⁴ is CₙH₂ₙNR⁵R⁶.

6. A compound according to claim 5 in which CₙH₂ₙNR⁵R⁶ is IV

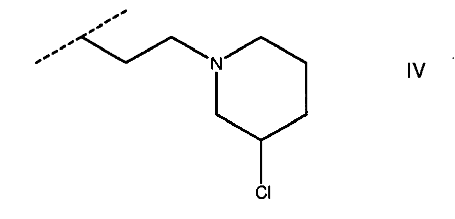

7. A compound according to claim 1 which is

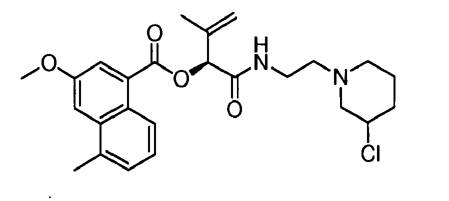

8. A compound according to any of claims 1-3 in which R³ is NH₂.

9. A compound according to claim 8 selected from

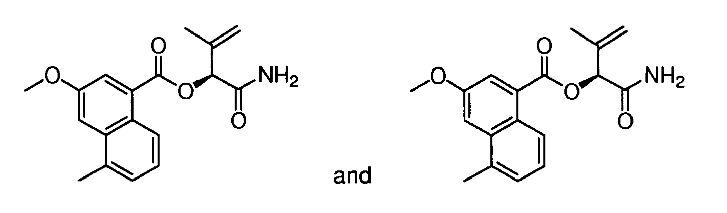

10. A compound according to any previous claim for use in a method of medical treatment of an animal by therapy.

11. Use of a compound according to any of claims 1-9 in the manufacture of a composition for use in a method of medical treatment of an animal by therapy, preferably in an anti-tumour treatment.

12. A pharmaceutical composition comprising the compound of any of claims 1-9 and a pharmaceutically acceptable excipient.

13. A synthetic method in which a compound of formula V 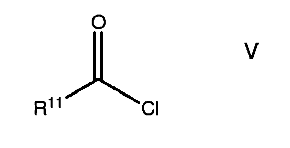 in which R¹¹ is selected from a group consisting of optionally substituted naphthyl, anthranyl and a group of formula III
is reacted with a compound of formula VI 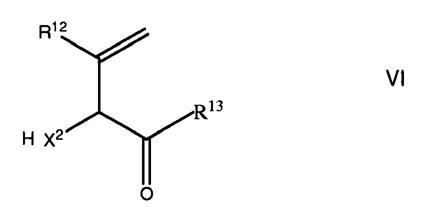 in which R¹² is H, optionally substituted C₁₋₄ alkyl, C₁₋₄ alkoxy, optionally substituted phenyl, C₇₋₁₂ aralkyl, optionally substituted heteroaryl or a ligand;
X² is O, NH or S;
R¹³ is OH, Cl, C₁₋₄ alkoxy or OPG wherein PG is a protecting group;
such that Cl in V is replaced in a nucleophilic substitution reaction by a group of formula VII 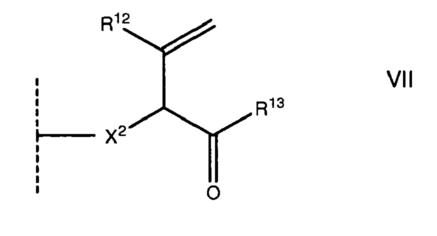 wherein the ligand is an oligopeptide, biotin, avidin, streptavidin, a polymeric group, an oligonucleotide or a protein.

14. A method according to claim 13 followed by removal of the protecting group to give R¹³ = OH.

15. A method according to claim 14 followed by reaction with a group of formula HR¹⁴ to give a compound of formula VIII 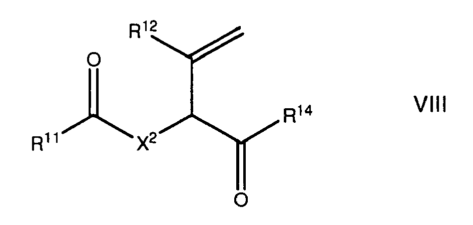 wherein R¹⁴ is selected from the group consisting of NH₂, NHR¹⁵, SR¹⁵ and OR¹⁵;
R¹⁵ is C₁₋₄ alkyl, C₁₋₄ substituted alkyl, C₁₋₄ alkoxy, optionally substituted phenyl, C₇₋₁₂ aralkyl, optionally substituted heteroraryl, CₚH₂ₚNR¹⁶R¹⁷ or a ligand;
in which at least one of R¹⁶ and R¹⁷ is (CH₂)₂A² or together with the nitrogen to which they are attached form a ring of formula IX 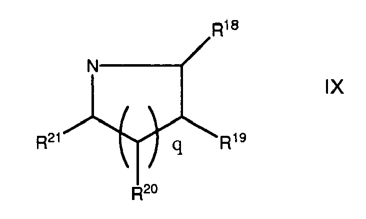 in which at least one of R¹⁸, R¹⁹ and R²⁰ is selected from A² and A² substituted C₁₋₄ alkyl, and any others are H or C₁₋₄ alkyl;
R²¹ is selected from H, C₁₋₄ alkyl, A² and A² substituted alkyl;
A² is a leaving group, halogen atom, hydroxyl or a protected hydroxyl;
q is 1-4;
p is 1-7;
wherein the substituent groups are selected from C₁₋₄ alkyl, hydroxyl, amino, alkyl amino, halo and aziridine.

16. A method according to any of claims 13-15 wherein the protecting group is benzyl.

17. A method according to any of claims 13-16 in which X² is O.

18. A method according to any of claims 13-17 in which R¹² is CH₃.

19. A method according to any of claims 13-18 in which the alkene to which R¹² is attached is oxidized to the corresponding epoxide.

20. A compound of general formula X 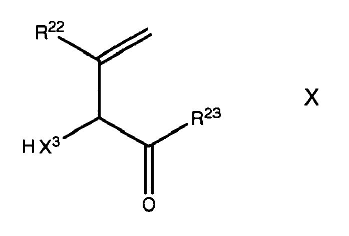 in which X³ is selected from the group consisting of O, NH and S;
R²² is H, C₁₋₄ alkyl, C₁₋₄ alkoxy, optionally substituted phenyl, C₇₋₁₂ aralkyl, optionally substituted heteroaryl or a ligand;
R²³ is a ligand or NHR²⁴ wherein R²⁴ is CᵣH₂ᵣNR²⁵R²⁶ or a ligand;
R²⁵ and R²⁶ are (CH₂)₂A³ or together with the nitrogen to which they are attached form a ring of formula XI 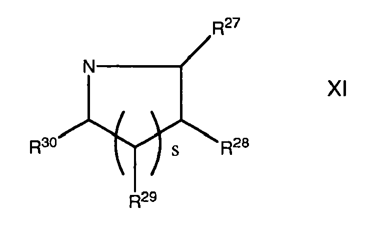 in which at least one of R²⁷, R²⁸ and R²⁹ is selected from A³ and A³ substituted C₁₋₄ alkyl and any others are H or C₁₋₄ alkyl, R³⁰ is selected from H, C₁₋₄ alkyl, A³ and A³ substituted C₁₋₄ alkyl;
A³ is a leaving group, halogen atom, hydroxyl or protected hydroxyl;
s is 1-4;
r is 1-7 and the ligand is an oligopeptide, biotin, avidin, streptavidin, a polymeric group, an oligonucleotide or a protein.

## Patentansprüche

1. Verbindung der Formel I oder ein Salz davon 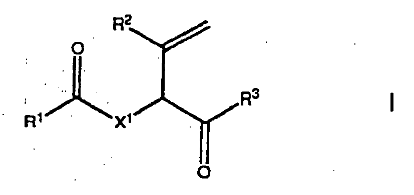 wobei X¹ ausgewählt ist aus einer Gruppe bestehend aus O, S und NR⁰, in der R⁰ H oder C₁₋₄-Alkyl ist;
R³ NH₂, NHR⁴, SR⁴, OR⁴, CH₂R⁴ oder OH ist;
R¹ gegebenenfalls substituiertes Naphthyl, Anthranyl oder eine Gruppe der Formel III ist 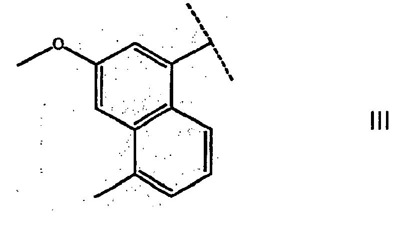 R² H, gegebenenfalls substituiertes C₁₋₄-Alkyl, C₁₋₄-Alkoxy, gegebenenfalls substituiertes Phenyl, C₇₋₁₂-Aralkyl, gegebenenfalls substituiertes Heteroaryl oder ein Ligand ist;
R⁴ C₁₋₄-Alkyl, C₁₋₄-substituiertes Alkyl, C₁₋₄-Alkoxy, gegebenenfalls substituiertes Phenyl, C₇₋₁₂-Aralkyl, gegebenenfalls substituiertes Heteroaryl, CₙH₂ₙNR⁵R⁶ oder ein Ligand ist;
wobei wenigstens einer der Reste R⁵ und R⁶ (CH₂)₂A¹ ist oder zusammen mit dem Stickstoff, an den er gebunden ist (sie gebunden sind), einen Ring der Formel II bildet (bilden) 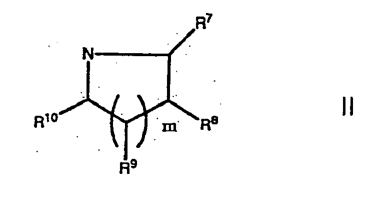 wobei wenigstens einer der Reste R⁷, R⁸ und R⁹ ausgewählt ist aus A¹ und mit A¹ substituiertem C₁₋₄-Alkyl und der andere Rest (die anderen Reste) H oder C₁₋₄-Alkyl ist (sind); R¹⁰ ausgewählt ist aus H, C₁₋₄-Alkyl, A¹ und mit A¹ substituiertem C₁₋₄-Alkyl;
A¹ eine austretende Gruppe oder ein Halogenatom ist;
m 1-4 ist;
n 1-7 ist;
wobei die Substituentengruppen ausgewählt sind aus C₁₋₄-Alkyl, Hydroxyl, Amino, Alkylamino, Halo und Aziridin und der Ligand ein Oligopeptid, Biotin, Avidin, Streptavidin, eine polymere Gruppe, ein Oligonukleotid oder ein Protein ist.

2. Verbindung nach Anspruch 1, in der X¹ O ist.

3. Verbindung nach Anspruch 1 oder 2, in der R² CH₃ ist.

4. Verbindung nach einem der Ansprüche 1-3, in der R³ NHR⁴ ist.

5. Verbindung nach Anspruch 4, in der R⁴ CₙH₂ₙNR⁵R⁶ ist.

6. Verbindung nach Anspruch 5, in der CₙH₂ₙNR⁵R⁶ IV ist.

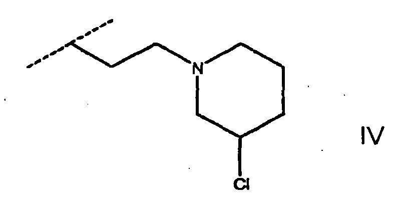

7. Verbindung nach Anspruch 1, nämlich

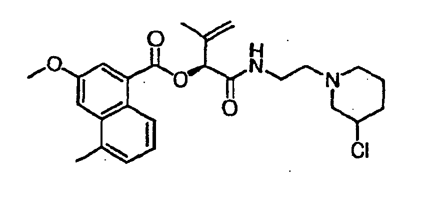

8. Verbindung nach einem der Ansprüche 1-3, in der R³ NH₂ ist.

9. Verbindung nach Anspruch 8, nämlich

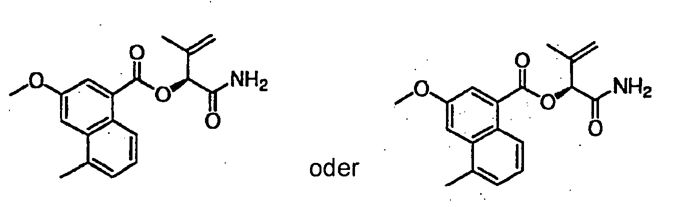

10. Verbindung nach einem der vorstehenden Ansprüche zur Verwendung bei einem Verfahren der therapeutischen medizinischen Behandlung eines Tiers.

11. Verwendung einer Verbindung nach einem der Ansprüche 1-9 bei der Herstellung einer Zusammensetzung zur Verwendung bei einem Verfahren der therapeutischen medizinischen Behandlung eines Tiers, vorzugsweise bei einer Anti-Tumor-Behandlung.

12. Pharmazeutische Zusammensetzung, umfassend eine Verbindung eines der Ansprüche 1-9 und ein pharmazeutisch verträgliches Bindemittel.

13. Syntheseverfahren, bei dem eine Verbindung der Formel V 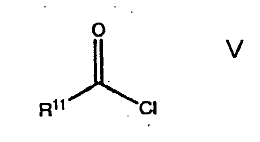 wobei R¹¹ ausgewählt ist aus einer Gruppe bestehend aus gegebenenfalls substituiertem Naphthyl, Anthranyl und einer Gruppe der Formel III,
umgesetzt wird mit einer Verbindung der Formel VI 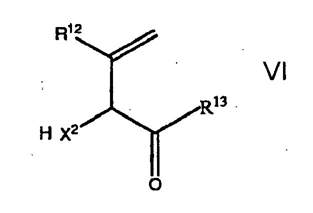 wobei R¹² H, gegebenenfalls substituiertes C₁₋₄-Alkyl, C₁₋₄-Alkoxy, gegebenenfalls substituiertes Phenyl, C₇₋₁₂-Aralkyl, gegebenenfalls substituiertes Heteroaryl oder ein Ligand ist;
X² O, NH oder S ist;
R¹³ OH, Cl, C₁₋₄-Alkoxy oder OPG ist, wobei PG eine Schutzgruppe bedeutet;
so dass Cl in V in einer nukleophilen Substitutionsreaktion durch eine Gruppe der Formel VII ersetzt wird 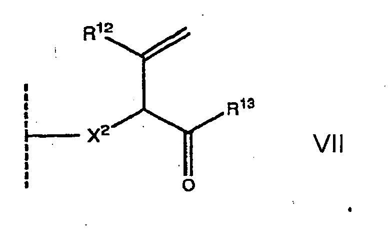 wobei der Ligand ein Oligopeptid, Biotin, Avidin, Streptavidin, eine polymere Gruppe, ein Oligonukleotid oder ein Protein ist.

14. Verfahren nach Anspruch 13, gefolgt von der Entfernung der Schutzgruppe zur Erzeugung von R¹³ = OH.

15. Verfahren nach Anspruch 14, gefolgt von der Reaktion mit einer Gruppe der Formel HR¹⁴ zur Erzeugung einer Verbindung der Formel VIII 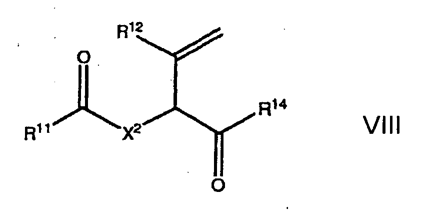 wobei R¹⁴ ausgewählt ist aus der Gruppe bestehend aus NH₂, NHR¹⁵, SR¹⁵ und OR¹⁵;
R¹⁵ C₁₋₄-Alkyl, C₁₋₄-substituiertes Alkyl, C₁₋₄-Alkoxy, gegebenenfalls substituiertes Phenyl, C₇₋₁₂-Aralkyl, gegebenenfalls substituiertes Heteroaryl, CₚH₂ₚNR¹⁶R¹⁷ oder ein Ligand ist;
wobei wenigstens einer der Reste R¹⁶ und R¹⁷ (CH₂)₂A² ist oder zusammen mit dem Stickstoff, an den er gebunden ist (sie gebunden sind), einen Ring der Formel IX bildet (bilden) 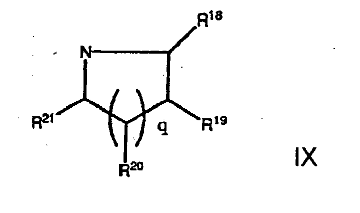 wobei wenigstens einer der Reste R¹⁸, R¹⁹ und R²⁰ ausgewählt ist aus A² und mit A² substituiertem C₁₋₄-Alkyl und der andere Rest (die anderen Reste) H oder C₁₋₄-Alkyl ist (sind);
R²¹ ausgewählt ist aus H, C₁₋₄-Alkyl, A² und mit A² substituiertem Alkyl;
A² eine austretende Gruppe, ein Halogenatom, Hydroxyl oder ein geschütztes Hydroxyl ist;
q 1-4 ist;
p 1-7 ist;
wobei die Substituentengruppen ausgewählt sind aus C₁₋₄-Alkyl, Hydroxyl, Amino, Alkylamino, Halo und Aziridin.

16. Verfahren nach einem der Ansprüche 13-15, wobei die Schutzgruppe Benzyl ist.

17. Verfahren nach einem der Ansprüche 13-16, bei dem X² O ist.

18. Verfahren nach einem der Ansprüche 13-17, bei dem R¹² CH₃ ist.

19. Verfahren nach einem der Ansprüche 13-18, bei dem das Alken, an das R¹² gebunden ist, zu dem entsprechenden Epoxid oxidiert wird.

20. Verbindung der allgemeinen Formel X 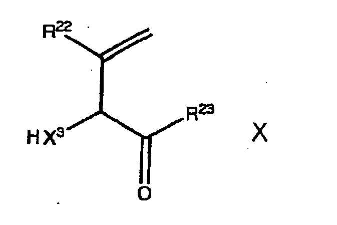 wobei X³ ausgewählt ist aus der Gruppe bestehend aus O, NH und S;
R²² H, C₁₋₄-Alkyl, C₁₋₄-Alkoxy, gegebenenfalls substituiertes Phenyl, C₇₋₁₂-Aralkyl, gegebenenfalls substituiertes Heteroaryl oder ein Ligand ist;
R²³ ein Ligand oder NHR²⁴ ist, wobei R²⁴ CᵣH₂ᵣNR²⁵R²⁶ oder ein Ligand ist;
R²⁵ und R²⁶ (CH₂)₂A³ sind oder zusammen mit dem Stickstoff, an den sie gebunden sind, einen Ring der Formel XI bilden 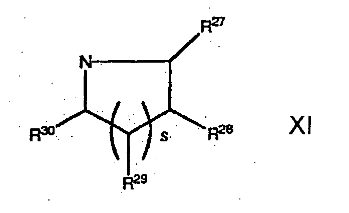 wobei wenigstens einer der Reste R²⁷, R²⁸ und R²⁸ ausgewählt ist aus A³ und mit A³ substituiertem C₁₋₄-Alkyl und der andere Rest (die anderen Reste) H oder C₁₋₄-Alkyl ist (sind), R³⁰ ausgewählt ist aus H, C₁₋₄-Alkyl, A³ und mit A³ substituiertem C₁₋₄-Alkyl ;
A³ eine austretende Gruppe, ein Halogenatom, Hydroxyl oder geschütztes Hydroxyl ist;
s 1-4 ist;
r 1-7 ist und der Ligand ein Oligopeptid, Biotin, Avidin, Streptavidin, eine polymere Gruppe, ein Oligonukleotid oder ein Protein ist.

## Revendications

1. Composé de formule I ou sel de celui-ci 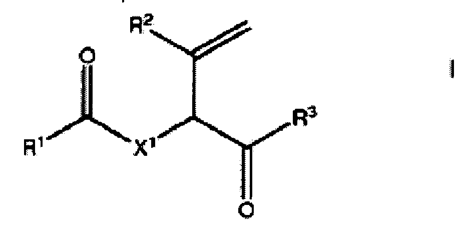 dans lequel X¹ est choisi dans le groupe constitué par O, S et NR⁰ où R⁰ est H ou un alkyle C₁₋₄;
R³ est NH₂, NHR⁴, SR⁴, OR⁴, CH₂R⁴ ou OH ;
R¹ est un naphtyle éventuellement substitué, un anthranyle, ou un groupe de formule III 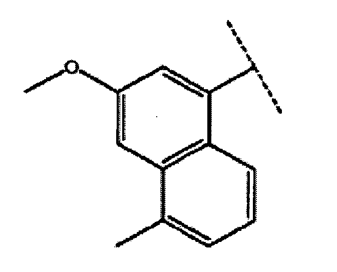 R² est H, un alkyle C₁₋₄ éventuellement substitué, un alcoxy C₁₋₄, un phényle éventuellement substitué, un aralkyle C₇₋₁₂, un hétéroaryle éventuellement substitué ou un ligand ;
R⁴ est un alkyle C₁₋₄, un alkyle C₁₋₄ substitué, un alcoxy C₁₋₄, un phényle éventuellement substitué, un aralkyle C₇₋₁₂, un hétéroaryle éventuellement substitué, CₙH₂ₙNR⁵R⁶ ou un ligand ;
au moins un des R⁵ et R⁶ étant (CH₂)₂A¹ ou ensemble avec l'atome d'azote auquel ils sont attachés, ils forment un cycle de formule II 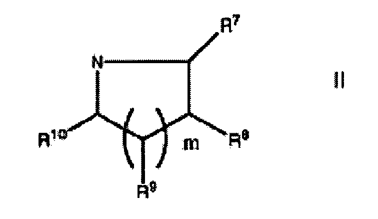 dans laquelle au moins un des R⁷, R⁸ et R⁹ est choisi parmi A¹ et un alkyle C₁₋₄ substitué par A¹ et les autres sont H ou un alkyle C₁₋₄ ; R¹⁰ est choisi parmi H, un alkyle C₁₋₄, A¹ et un alkyle C₁₋₄ substitué par A¹;
A¹ est un groupe labile ou un atome d'halogène ;
m vaut de 1 à 4 ;
n vaut de 1 à 7 ;
les groupes substituants étant choisis parmi un alkyle C₁₋₄, un hydroxyle, amino, alkylamino, un atome d'halogène et une aziridine et le ligand est un oligopeptide, la biotine, l'avidine, la streptavidine, un groupe polymère, un oligonucléotide ou une protéine.

2. Composé selon la revendication 1, dans lequel X¹ est O.

3. Composé selon la revendication 1 ou la revendication 2, dans lequel R² est CH₃.

4. Composé selon l'une quelconque des revendications 1 à 3, dans lequel R³ est NHR⁴.

5. Composé selon la revendication 4, dans lequel R⁴ est CₙH₂ₙNR⁵R⁶_{.}

6. Composé selon la revendication 5, dans lequel CₙH₂ₙNR⁵R⁶ est IV

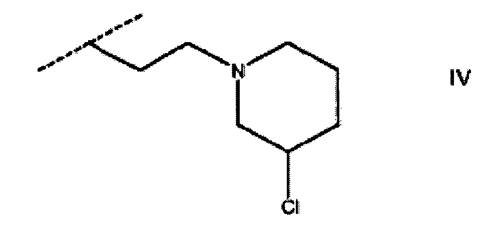

7. Composé selon la revendication 1 qui est

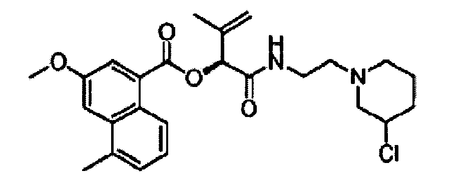

8. Composé selon l'une quelconque des revendications 1 à 3, dans lequel R³ est NH₂.

9. Composé selon la revendication 8 choisi parmi

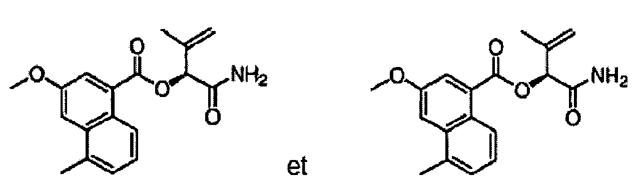

10. Composé selon l'une quelconque des revendications précédentes utilisable dans un procédé de traitement médical d'un animal par thérapie.

11. Utilisation d'un composé selon l'une quelconque des revendications 1 à 9 dans la fabrication d'une composition utilisable dans un procédé de traitement médical d'un animal par thérapie, de préférence, dans un traitement antitumoral.

12. Composition pharmaceutique comprenant le composé selon l'une quelconque des revendications 1 à 9 et un excipient pharmaceutiquement acceptable.

13. Procédé de synthèse, dans lequel un composé de formule V 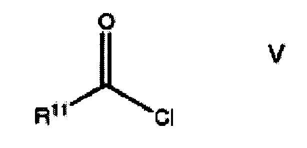 dans laquelle R¹¹ est choisi dans le groupe constitué par un naphtyle éventuellement substitué, un anthranyle et un groupe de formule III
est mis en réaction avec un composé de formule VI 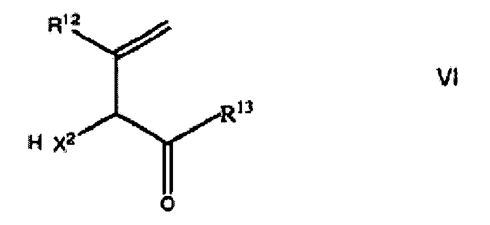 dans laquelle R¹² est H, un alkyle C₁₋₄ éventuellement substitué, un alcoxy C₁₋₄, un phényle éventuellement substitué, un aralkyle C₇₋₁₂, un hétéroaryle éventuellement substitué ou un ligand ;
X² est O, NH ou S ;
R¹³ est OH, Cl, un alcoxy C₁₋₄ ou OPG où PG est un groupe de protection ;
de façon que Cl dans V soit remplacé dans une réaction de substitution nucléophile par un groupe de formule VII 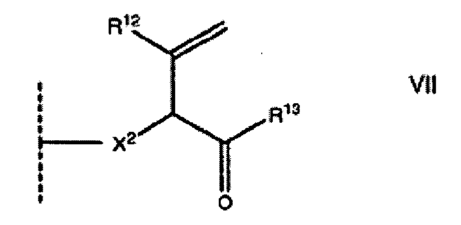 dans laquelle le ligand est un oligopeptide, la biotine, l'avidine, la streptavidine, un groupe polymère, un oligonucléotide ou une protéine.

14. Procédé selon la revendication 13, suivi par l'élimination du groupe de protection pour obtenir R¹³ = OH.

15. Procédé selon la revendication 14, suivi par la réaction avec un groupe de formule HR¹⁴ pour obtenir un composé de formule VIII 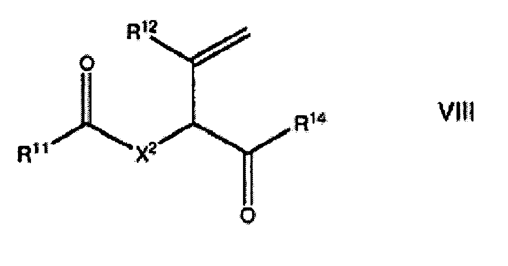 dans laquelle R¹⁴ est choisi dans le groupe constitué par NH₂, NHR¹⁵ SR¹⁵ et OR¹⁵;
R¹⁵ est un alkyle C₁₋₄, un alkyle C₁₋₄ substitué, un alcoxy C₁₋₄, un phényle éventuellement substitué, un aralkyle C₇₋₁₂, un hétéroaryle éventuellement substitué, CₚH₂ₚNR¹⁶R¹⁷ ou un ligand ;
au moins un des R¹⁶ et R¹⁷ étant (CH₂)₂A² ou ensemble avec l'atome d'azote auquel ils sont attachés, ils forment un cycle de formule IX 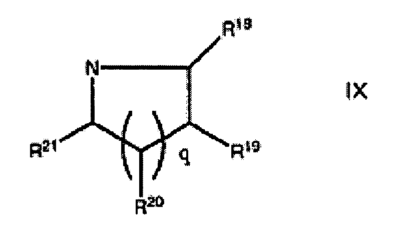 dans laquelle au moins un des R¹⁸, R¹⁹ et R²⁰ est choisi parmi A² et un alkyle C₁₋₄ substitué par A² et les autres sont H ou un alkyle C₁₋₄;
R²¹ est choisi parmi H, un alkyle C₁₋₄, A² et un alkyle substitué par A²;
A² est un groupe labile, un atome d'halogène, un hydroxyle ou un hydroxyle protégé ;
q vaut de 1 à 4;
p vaut de 1 à 7;
les groupes substituants étant choisis parmi un alkyle C₁₋₄, un hydroxyle, amino, alkylamino, un atome d'halogène et une aziridine.

16. Procédé selon l'une quelconque des revendications 13 à 15, dans lequel le groupe de protection est un benzyle.

17. Procédé selon l'une quelconque des revendications 13 à 16, dans lequel X² est O.

18. Procédé selon l'une quelconque des revendications 13 à 17, dans lequel R¹² est CH₃.

19. Procédé selon l'une quelconque des revendications 13 à 18, dans lequel l'alcène auquel R¹² est attaché est oxydé pour obtenir l'époxyde correspondant.

20. Composé de formule générale X 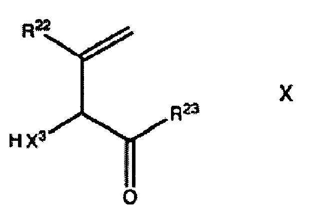 dans laquelle X³ est choisi dans le groupe constitué par O, NH et S;
R²² est H, un alkyle C₁₋₄, un alcoxy C₁₋₄, un phényle éventuellement substitué, un aralkyle C₇₋₁₂, un hétéroaryle éventuellement substitué ou un ligand ;
R²³ est un ligand ou NHR²⁴, où R²⁴ est CᵣH₂ᵣNR²⁵R²⁶ ou ligand;
R²⁵ et R²⁶ sont (CH₂)₂A³ ou ensemble avec l'atome d'azote auquel ils sont attachés, ils forment un cycle de formule XI 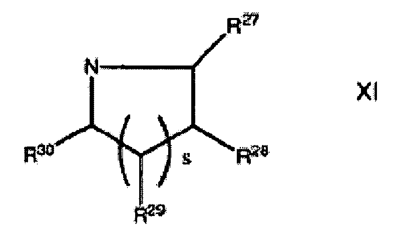 dans laquelle au moins un des R²⁷, R²⁸ et R²⁹ est choisi parmi A³ et un alkyle C₁₋₄ substitué par A³ et les autres sont H ou un alkyle C₁₋₄, R³⁰ est choisi parmi H, un alkyle C₁₋₄, A³ et un alkyle C₁₋₄ substitué par A³ ;
A³ est un groupe labile, un atome d'halogène, un hydroxyle ou un hydroxyle protégé ;
s vaut de 1 à 4;
r vaut de 1 à 7 et le ligand est un oligopeptide, la biotine, l'avidine, la streptavidine, un groupe polymère, un oligonucléotide ou une protéine.
